(19) Europäisches Patentamt
European Patent Office
Office européen des brevets

(11) Publication number: **0 284 030 B1**

(12) # EUROPEAN PATENT SPECIFICATION

(45) Date of publication of patent specification: **17.03.93**

(51) Int. Cl.5: **C07D 231/22**, C07D 401/04, A01N 43/56

(21) Application number: **88104584.3**

(22) Date of filing: **22.03.88**

(54) **Pyrazole derivative, process thereof and herbicide containing the derivative as an effective component.**

(30) Priority: **23.03.87 JP 68477/87**

(43) Date of publication of application: **28.09.88 Bulletin 88/39**

(45) Publication of the grant of the patent: **17.03.93 Bulletin 93/11**

(84) Designated Contracting States: **DE FR GB IT**

(56) References cited: **DE-A- 3 625 686**

(73) Proprietor: **Otsuka Kagaku Kabushiki Kaisha
10, Bungo-machi Higashi-ku
Osaka-shi Osaka-fu(JP)**

(72) Inventor: **Endo, Yoshinori c/o Otsuka Kagaku Kabushiki Kaisha
649-2, Satoura aza Hanamen
Satoura-cho Naruto-shi Tokushima-ken(JP)**

(74) Representative: **Barz, Peter, Dr. et al
Patentanwälte Dipl.-Ing. G. Dannenberg Dr. P. Weinhold, Dr. D. Gudel Dipl.-Ing. S. Schubert, Dr. P. Barz Siegfriedstrasse 8
W-8000 München 40 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.5x/3.0.1)

## Description

The present invention relates to a pyrazole derivative, process thereof and herbicide containing the derivative as an effective component.

Pyrazole derivatives are known to have herbicidal action and as 4-nitropyrazole derivatives are disclosed 4-nitro-5-propionamide-1-(2,3,4-trichlorophenyl)pyrazole and the like in Japanese Tokkyo Kokai No. 174,756/1985. These pyrazole derivatives have a group derived from an amino derivative at 5-position of the pyrazole ring. The above Kokai document is quite silent about pyrazole derivatives having a group derived from an oxy derivative at 5-position of the pyrazole ring. Further, the pyrazole derivatives disclosed in the above Kokai document are not sufficient in both of herbicidal action and crop selectivitiy.

An object of the invention is to provide a novel pyrazole derivative which is excellent in herbicidal action and crop selectivity.

The above and other objects of the invention will become apparent from the following description.

The pyrazole derivative of the invention is a novel compound which is not disclosed in the prior art and is represented by the following formula (I)

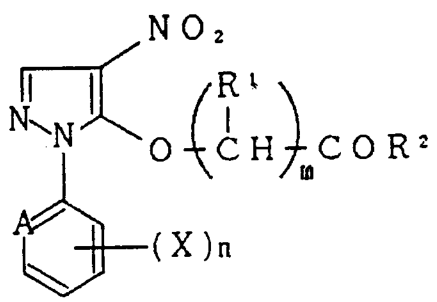

(I)

wherein $R^1$ is hydrogen atom or lower alkyl, $R^2$ is hydroxyl, $-OR^3$, $-SR^3$ or $-NR^4R^5$, $R^3$ is alkyl, lower alkenyl, lower alkynyl, cycloalkyl, lower alkoxyalkyl, substituted or unsubstituted phenyl or substituted or unsubstituted benzyl, $R^4$ and $R^5$ are same or different and are hydrogen atom, lower alkyl, lower alkenyl, lower alkynyl, hydroxyl, lower alkoxyl, lower alkyl having substituent(s) selected from the group consisting of halogen atom, cyano, hydroxyl and alkoxyl, cycloalkyl, substituted or unsubstituted phenyl or substituted or unsubstituted benzyl, $R^4$ and $R^5$ may link together to form 5 or 6-membered ring, X is halogen atom, trifluoromethyl, trifluoromethoxy or trifluoromethylthio, ⋗A represents ⋗CH or ⋗N, m is 1 or 2, n is an integer of 1 to 5.

The present pyrazole derivative of the formula (I) has an excellent herbicidal activity and is useful as a herbicide. As shown later, the present pyrazole derivative has excellent features that it exhibits high herbicidal activity in both of fields and paddy fields, and hardly influences on crops.

In the present specification, examples of alkyl groups are methyl, ethyl, n-propyl, isopropyl, n-butyl, isobutyl, sec-butyl, tert-butyl, n-pentyl, isopentyl, n-hexyl, isohexyl, n-octyl, n-decyl and like alkyls having 1 to 12 carbon atoms.

Examples of lower alkenyl groups are those having 2 to 6 carbon atoms such as vinyl, propenyl and butenyl.

Lower alkynyl groups include ethynyl, propynyl, butynyl and like alkynyls having 2 to 6 carbon atoms.

Lower alkoxyalkyl groups include those having 2 to 8 carbon atoms such as methoxymethyl, methoxyethyl, methoxypropyl, ethoxymethyl, butoxymethyl and methoxybutyl.

Examples of substituents of phenyl and benzyl groups are halogen atom, alkyl of 1 to 3 carbon atoms, alkoxyl of 1 to 3 carbon atoms, nitro and cyano.

Halogen atoms include fluorine, chlorine and bromine atoms.

Lower alkyl groups include those having 1 to 6 carbon atoms such as methyl, ethyl, n-propyl, isopropyl, n-butyl, isobutyl, sec-butyl, tert-butyl, n-pentyl, isopentyl, n-hexyl and isohexyl.

Examples of lower alkoxyl groups are methoxy, ethoxy, propoxy, butoxy and like alkoxyl of 1 to 6 carbon atoms.

Lower alkyl groups having substituent(s) selected from the group consisting of halogen atom, cyano, hydroxyl and alkoxyl include substituted alkyls having 1 to 6 carbon atoms such as chloromethyl, bromomethyl fluoromethyl, 1-chloroethyl, 2-chloroethyl, 1-bromoethyl, 2-bromoethyl, 1-fluoroethyl, 2-fluoroethyl, 1-chloropropyl, 2-chloropropyl, 3-chloropropyl, 1-bromopropyl, 2-bromopropyl, 3-bromopropyl,

1,1-dichloroethyl, 1,2-dichloroethyl, 2,2-dichloroethyl, 1,1-dibromoethyl, 1,2-dibromoethyl, 2,2-dibromoethyl, hydroxymethyl, 1-hydroxyethyl, 2-hydroxyethyl, 1-hydroxypropyl, 2-hydroxypropyl, 3-hydroxypropyl, cyanomethyl, 1-cyanoethyl, 2-cyanoethyl, 1-cyanopropyl, 2-cyanopropyl, 3-cyanopropyl, methoxymethyl, 1-methoxyethyl, 2-methoxyethyl, 1-methoxypropyl, 2-methoxypropyl, 3-methoxypropyl, ethoxymethyl, 1-ethoxyethyl, 2-ethoxyethyl, butoxymethyl, 1-butoxyethyl and 2-butoxyethyl.

Examples of cycloalkyl groups are those having 3 to 8 carbon atoms such as cyclopropyl, cyclopentyl and cyclohexyl.

Examples of 5 to 6 membered rings formed by combination of $R^4$ and $R^5$ are heterocyclic rings such as pyrrole, pyrazole, triazole, imidazole, morpholino and piperazino.

The present compound can be prepared by various methods and can easily be prepared in general by the following reaction equation 1, 2 or 3.

Reaction equation 1 :

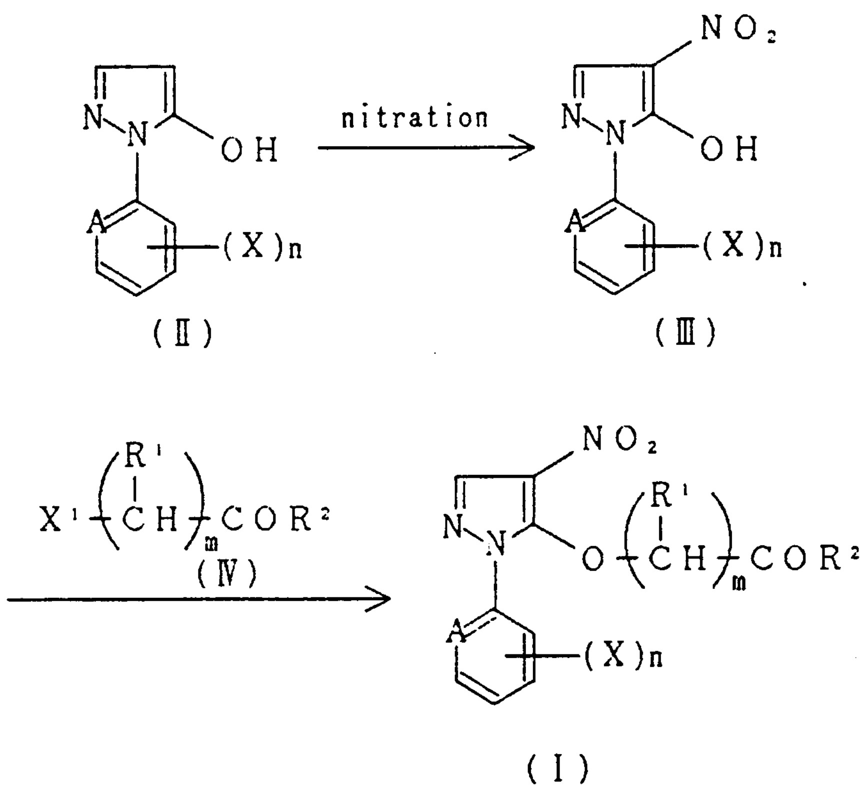

Reaction equation 2 :

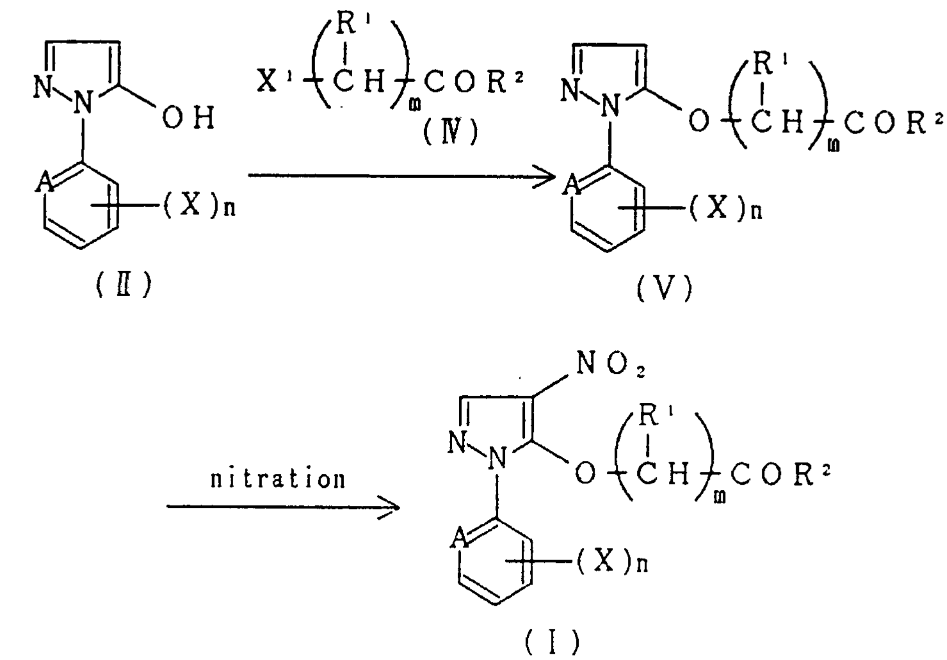


(II) (V) (I)

Reaction equation 3 :

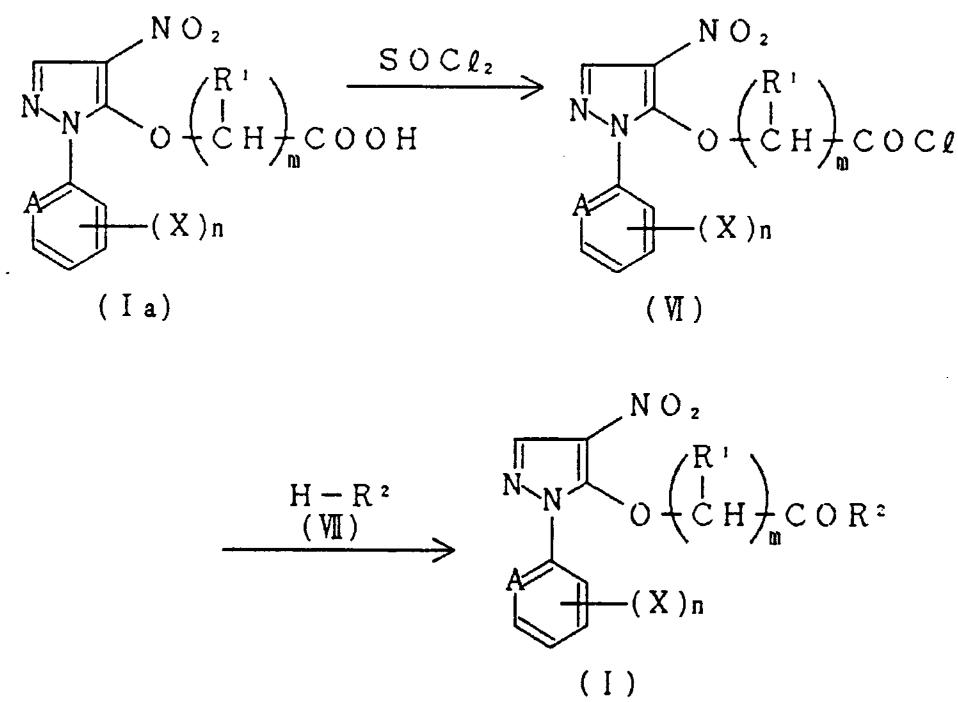


(Ia) (VI) (I)

In the above, $R^1$, $R^2$, X, ⋗A, m and n are the same as above, $X^1$ is halogen atom.

According to Reaction equation 1, the present compound (I) is prepared by nitrating a pyrazole derivative (II) and reacting a compound (IV) with the resulting pyrazole derivative (III).

The nitration of the compound (II) is conducted in the same manner as usual nitration of an aromatic ring. Examples of nitration reagents are conc. nitric acid, fuming nitric acid, sodium nitrate and potassium

nitrate. Solvents and catalysts include acetic acid, acetic anhydride and conc. sulfuric acid. The amount of the nitration reagent is not particularly limited and is usually about 1 to 10 moles per mole of the compound (II). The reaction proceeds at a temperature usually of -10 ° C to room temperature and the reaction time is generally about 0.5 to 5 hours.

The reaction of the compound (III) and the compound (IV) is conducted in an appropriate solvent. Examples of solvents are diethyl ether, dibutyl ether, tetrahydrofuran, dioxane and like ethers, methylene chloride, chloroform, dichloroethane, carbon tetrachloride and like halogenated hydrocarbons, acetone, methyl ethyl ketone, methyl isobutyl ketone, cyclohexanone and like ketones, dimethyl formamide and dimethyl sulfoxide. The prportions of the compound (III) and the compound (IV) is not particularly limited and the latter is used usually in an amount of about 0.5 to 2 moles, preferably 0.7 to 1.5 moles per mole of the former. In the reaction, a basic compound is preferably used. The basic compound is any of conventional one which scavenges hydrogen halide resulting from the reaction and includes sodium carbonate, potassium carbonate, sodium hydroxide, potassium hydroxide, sodium metal and sodium hydride. The amount of the basic compound is usually about 0.5 to 3 moles, preferably 0.7 to 2 moles per mole of the compound (III). The reaction proceeds preferably at a temperature from room temperature to a boiling point of the solvent used and the reaction time is usually about 1 to 15 hours.

According to Reaction equation 2, the present compound (I) is prepared by reacting the pyrazole derivative (II) with the compound (IV) and nitrating the resulting pyrazole derivative (V).

The reaction of the compound (II) and the compound (IV) is conducted without a solvent or in an appropriate solvent. Examples of solvents are diethyl ether, dibutyl ether, tetrahydrofuran, dioxane and like ethers, methylene chloride, chloroform, dichloroethane, carbon tetrachloride and like halogenated hydrocarbons, acetone, methyl ethyl ketone, methyl isobutyl ketone, cyclohexanone and like ketones, methanol, ethanol and like alcohols, dimethyl formamide, dimethyl sulfoxide and water. The prportions of the compound (II) and the compound (IV) is not particularly limited and the latter is used usually in an amount of about 0.5 to 2 moles, preferably 0.7 to 1.5 moles per mole of the former. In the reaction, a basic compound is preferably used. The basic compound is any of conventional one which scavenges hydrogen halide resulting from the reaction and includes sodium carbonate, potassium carbonate, sodium hydroxide, potassium hydroxide, sodium metal, sodium methylate, sodium ethylate and like sodium alcoholates, sodium hydride, triethylamine, tributylamine, dimethylaniline, diethylaniline and like tertiary amines. The amount of the basic compound is usually about 0.5 to 2 moles, preferably 0.7 to 1.5 moles per mole of the compound (II). The reaction proceeds at room temperature or with heating and preferably from room temperature to 150 ° C and the reaction time is generally about 0.5 to 5 hours.

The nitration of the obtained compound (V) is conducted in the same manner as in the nitration of the compound (II) in Reaction equation 1. In case of using the compound wherein $R^2$ is hydroxyl group, dehydration may occur between two molecules to form carboxylic anhydride, which is however easily hydrated to form the compound (I).

In accordance with Reaction equation 3, the compound (I) is prepared by reacting thionyl chloride with the pyrazole derivative (Ia) which is obtained in the above Reaction equation 1 or 2, and reacting the resulting acid chloride (VI) with the compound (VII).

The reaction of the compound (Ia) and thionyl chloride is conducted with or without a solvent. The solvent includes benzene, toluene, xylene and like aromatic hydrocarbons, methylene chloride, chloroform, dichloroethane, carbon tetrachloride and like halogenated hydrocarbons. The proportion of the compound (Ia) and thionyl chloride is not particularly limited but the latter is used usually about 0.5 to 10 moles, preferably about 1.5 to 8 moles per mole of the former. The above reaction is carried out generally at 50 to 150 ° C and the reaction time is generally about 1 to 10 hours. The obtained compound (VI) is used for the following reaction with or without isolation.

The reaction of the compound (VI) and the compound (VII) is carried out with or without a solvent. Examples of solvents are diethyl ether, dibutyl ether, tetrahydrofuran, dioxane and like ethers, methylene chloride, chloroform, dichloroethane, carbon tetrachloride and like halogenated hydrocarbons, benzene, toluene, xylene and like aromatic hydrocarbons. The proportion of the compound (VI) and the compound (VII) is not particularly limited but the latter is used usually about 0.5 to 5 moles, preferably about 0.7 to 4 moles per mole of the former. In the above reaction is preferably used a basic compound. The basic compound is any of conventional one which scavenges hydrogen chloride resulting from the reaction and includes triethylamine, tributylamine, dimethylaniline, diethylaniline and like tertiary amines, pyridine, picoline and like pyridines. In case the compound (VII) is an amine derivative, the compound is used as the basic compound. The basic compound is used in an amount of usually about 0.5 to 5 moles, preferably about 0.7 to 4 moles per mole of the compound (VI). The above reaction is conducted at room temperature or with heating and proceeds usually at room temperature to 100 ° C. The reaction time is usually about 1 to

10 hours.

The present compound obtained by the above method is easily isolated from the reaction mixture and produced in a high purity by a usual isolation means such as solvent extraction, solvent dilution, recrystallization and column chromatography.

The present compound has excellent characteristics that a high herbicidal action is exhibited in upland fields and paddy fields but on the other hand, hardly affect crops. The compounds of the present invention exhibit a controlling action against preemergence or postemergence: they are particularly useful as herbicides in the upland field against pigweed, smortweed, barnyardgrass, common purslane, hairy galinsoga, large crabgrass, gosaegrass, foxtail, flatsedge, redroot pigweed, velvetleaf, jimsonweed, Johnsongrass, etc.; in the paddy field against weeds such as common falsepimpernel, toothcup, barnyardgrass, flatsedge, Monochoria vaginalis, bulrush, Cyperus serotinus, Sagittaria pygmaea, etc. On the other hand, the compounds of the present invention show good tolerance to crops such as rice, soybean, sunflower, cotton, wheat, corn, etc. and can be used as selective herbicides.

As described above, the compounds of the present invention have a very wide herbicidal spectrum and are safe to crops and therefore, can be effectively used to control weeds in paddy fields, upland fields for various vegetables, crops and fruits mulberry field and non-cropland, etc.

For applying the compounds of the present invention as herbicides, they may be used as they are but are generally used in admixture with adjuvants conventionally used to make up agricultural chemicals into a form convenient to use. The form is not particularly limited but preferred are powders, emulsions, wettable powders, flowables and granules. As the adjuvants, those conventionally used in the art may be widely used and mention may be made of, for example, thickeners such as kieselguhr, kaolin, clay, bentonite, white carbon and talc; surface active agents such as polyoxyethylene alkyl ethers, polyoxyethylene alkylphenyl ethers, polyoxyethylene sorbitan fatty acid ethers, polyoxyethylene fatty acid ethers, sodium alkylbenzenesulfonates, sodium ligninsulfonates, sodium alkylsulfates and sodium polyoxyethylene alkylsulfates; organic solvents such as benzene, toluene, xylene, acetone, cyclohexanone, methanol, ethanol, isopropyl alcohol, dioxane, dimethylformamide, dimethyl sulfoxide and carbon tetrachloride.

The amount of the effective component used in the herbicide of the present invention is not particularly limited but it is preferable to add the adjuvant in such that usually about 0.1 to 90% by weight, preferably about 1 to 70% by weight of the effective component is contained in the herbicide.

Upon use of the herbicidal composition of the present invention, the herbicidal agent of the present invention may be sprinkled without dilution as it is, or may also be diluted to approximately 500 to 5000 times and the dilution may be sprinkled. The dose applied may vary depending upon preparation form of chemicals, method for application, time for application, weeds to be controlled, etc. and has no general limit but it is generally preferred that the effective component be 0.01 to 5 kg/ha, preferably 0.05 to 2 kg/ha, in the case of using in paddy fields, and in the case of using in cultivated fields, 0.01 to 5 kg/ha, preferably 0.05 to 3 kg/ha.

The invention will be described in more detail with reference to examples, herbicide preparation and test examples.

## Example 1

Preparation of 5-hydroxy-4-nitro-1-(2,3,4-trichlorophenyl)pyrazole

To 50ml of glacial acetic acid were added dropwise 2.3g of acetic anhydride and 2.3g of conc. nitric acid (61%) with ice-cooling and stirring. To the mixture was added 5g of 1-(2,3,4-trichlorophenyl)-5-pyrazolone in a small feed rate with ice-cooling (0~5 °C) and the mixture was further stirred for 2 hours with stirring. The mixture was poured into 100g of ice-water and the resulting precipitates were filtered and washed with water. A solution of the precipitates in 20ml of methanol was poured into 50ml of aqueous solution of 2N sodium hydroxide. The solution was washed twice with 30ml of ether. The aqueous layer was made acidic with addition of conc. hydrochloric acid and extracted twice with 50ml of chloroform. The chloroform layer was washed with saturated aqueous solution of sodium chloride and dried over anhydrous magnesium sulfate. Solvent was removed at a reduced pressure to obtain an oil. The oil was dissolved in 50ml of benzene and the insolubles were removed by filtration. Benzene was removed at a reduced pressure and the obtained oil was crystallized from benzene and hexane. The crystal was filtered to obtain 1.6g of the desired product.

m. p. 159~160 °C

NMR δppm (DMSO-$d_6$)

7.1~7.7 (2H, aromatic ring)

7.97 (s, 1H)

Example 2

Preparation of methyl [4-nitro-1-(2,3,4-trichlorophenyl)pyrazole-5-yl]-2-propionate

To 10ml of methyl ethyl ketone were added 1g of 5-hydroxy-4-nitro-1-(2,3,4-trichlorophenyl)pyrazole and 0.45g of anhydrous potassium carbonate. The mixture was refluxed with heating and stirring for 30 minutes. To the mixture was added 0.7g of methyl α-bromopropionate and the mixture was refluxed with heating and stirring for 72 hours. To the residue obtained by removing the solvent at a reduced pressure were added 50ml of ether and 30ml of water. The ether layer was washed with saturated aqueous solution of sodium chloride and then dried over anhydrous magnesium sulfate. The oil obtained by removing the solvent at a reduced pressure was purified by silica gel column chromatography to obtain 0.12g of the desired product.

m. p. 101~102°C
NMR δppm ($CDCl_3$)
1.55 (d, 3H)
3.58 (s, 3H)
5.47 (q, 1H)
7.0~7.6 (2H, aromatic ring)
8.06 (s, 1H)

| Elementary analysis ($C_{13}H_{10}N_3O_5Cl_3$ : 394.606) | | | |
|---|---|---|---|
| Calcd (%) | C : 39.57 | H : 2.55 | N : 10.65 |
| Found (%) | C : 39.11 | H : 3.02 | N : 10.55 |

From the above, the following compound was confirmed.

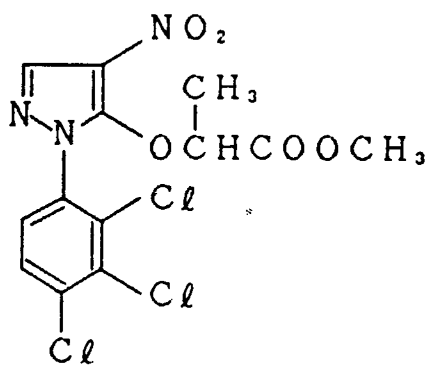

Example 3

Preparation of ethyl [1-(2,3,4-trichlorophenyl)pyrazole-5-yl]-2-propionate

To a mixture of 50ml of dimethyl formamide (DMF) and 10g of 1-(2,3,4-trichlorophenyl)-5-pyrazolone was added 1.7g of 60% oily sodium hydride in a small feed rate with ice-cooling and stirring and the mixture was stirred with ice-cooling for 30 minutes. To the mixture was added dropwise 7.6g of ethyl α-bromopropionate with ice-cooling and stirred at room temperature for 30 minutes. The mixture was poured into 200g of ice-water and extracted twice with 200ml of ether. The ether solution was washed with saturated aqueous solution of sodium chloride and dried over anhydrous magnesium sulfate. The oil obtained by removing the solvent at a reduced pressure was purified by silica gel column chromatography (developer ; hexane/ethyl acetate =4/1) to obtain 9.5g of the oily desired product.

m. p. 101~102°C
NMR δppm ($CDCl_3$)

1.19 (t, 3H)
1.45 (d, 3H)
4.08 (q, 2H)
4.58 (q, 1H)
5.42 (d, 1H)
7.0~7.5 (2H, aromatic ring, and 1H, pyrazole 3-position)

Example 4

Preparation of ethyl [4-nitro-1-(2,3,4-trichlorophenyl)pyrazole-5-yl]-2-propionate

To a mixture of 5.6g of acetic anhydride and 1.0g of ethyl [1-(2,3,4-trichlorophenyl)pyrazole-5-yl]-propionate was added dropwise 4.3g of fuming nitric acid at 20 to 30° C with stirring and the mixture was further stirred at room temperature for 30 minutes. The mixture was poured into 50g of ice-water and extracted twice with 50ml of ether. The ether solution was washed with water, sodium hydrogencarbonate aqueous solution, saturated sodium chloride aqueous solution and dried over anhydrous magnesium sulfate. The solvent was removed at a reduced pressure and the resulting oil was purified by silica gel column chromatography to obtain 0.65g of the desired product.

m. p. 91~92° C
NMR δppm ($CDCl_3$)
1.20 (t, 3H)
1.55 (d, 3H)
4.06 (q, 2H)
5.50 (q, 1H)
7.0~7.7 (2H, aromatic ring)
8.06 (s, 1H)

| Elementary analysis ($C_{14}H_{12}N_3O_5Cl_3$ : 408.633) | | | |
|---|---|---|---|
| Calcd (%)<br>Found (%) | C : 41.15<br>C : 41.66 | H : 2.96<br>H : 3.09 | N : 10.28<br>N : 10.11 |

From the above, the following compound was confirmed.

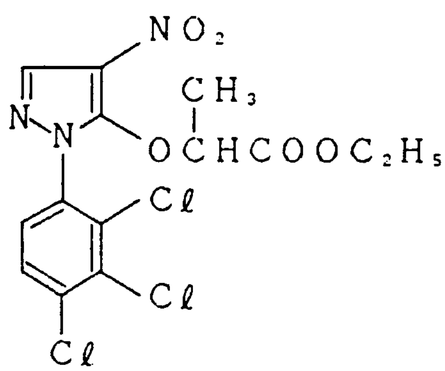

Example 5

Preparation of [1-(2,3,4-trichlorophenyl)pyrazole-5-yl]-2-propionic acid

To a mixture of 200ml of 90% ethanol and 9.5g of ethyl [1-(2,3,4-trichlorophenyl)pyrazole-5-yl]-2-propionate was added 2.3g of sodium hydroxide and the mixture was refluxed with heating for 3 to 4 hours until the starting material disappeared by thin layer chromatography (TLC). The residue obtained by removing the solvent at a reduced pressure was dissolved in 200g of ice-water and washed twice with 100ml of ether. The aqueous solution was made acidic with addition of conc. hydrochloric acid and

extracted twice with 100ml of chloroform. The chloroform solution was dried over anhydrous magnesium sulfate and the solvent was removed at a reduced pressure to obtain an oily residue. The desired product (8.5g) was obtained by crystallization from benzene and hexane and washing with hexane.

m. p. 168~169 °C

NMR δppm (DMSO-$d_6$)

1.49 (d, 3H)

4.12 (q, 1H)

5.56 (d, 1H)

7.37 (d, 1H)

7.1~7.6 (2H, aromatic ring)

7.83 (broad, 1H)

Example 6

Preparation of thioethyl [4-nitro-1-(2,3,4-trichlorophenyl)pyrazole-5-yl]-2-propionate

To a mixture of 6g of acetic anhydride and 2g of [1-(2,3,4-trichlorophenyl)pyrazole-5-yl]-2-propionic acid was added dropwise 5.6g of fuming nitric acid with ice-cooling and stirring, maintaining the reaction temperature at 5 to 10 °C. After completion of the addition, the mixture was poured into 100g of ice-water and extracted with 100ml of ether. The ether solution was extracted twice with 100 ml of aqueous solution of 2N sodium hydroxide. The aqueous solution was made acidic with addition of conc. hydrochloric acid and extracted twice with 50ml of chloroform. The extract was dried over anhydrous magnesium sulfate and the solvent was removed at a reduced pressure to obtain 0.9g of viscous oil. To the oil was added 5ml of thionyl chloride and the mixture was refluxed with heating for 1 hour. Excess of thionyl chloride was removed at a reduced pressure to obtain a crude product of oily acid chloride. The product was dissolved in 20ml of benzene without purification and 0.37g of ethyl mercaptan was added. To the mixture was added dropwise 0.5g of pyridine with ice-cooling and stirring and stirred at room temperature for further 1 hour. The mixture was washed with 2N hydrochloric acid aqueous solution, water and saturated sodium chloride aqueous solution, and dried over anhydrous magnesium sulfate. The solvent was removed at a reduced pressure and the resulting oil was purified by silica gel column chromatography to obtain 0.45g of oily desired product.

NMR δppm ($CDC\ell_3$)

1.15 (t, 3H)

1.49 (d, 3H)

2.75 (q, 2H)

5.41 (q, 1H)

7.1~7.6 (2H, aromatic ring)

8.04 (s, 1H)

| Elementary analysis ($C_{14}H_{12}N_3O_4SC\ell_3$ : 424.697) | | | |
|---|---|---|---|
| Calcd (%) | C : 39.59 | H : 2.85 | N : 9.90 |
| Found (%) | C : 39.98 | H : 2.76 | N : 9.65 |

From the above, the following compound was confirmed.

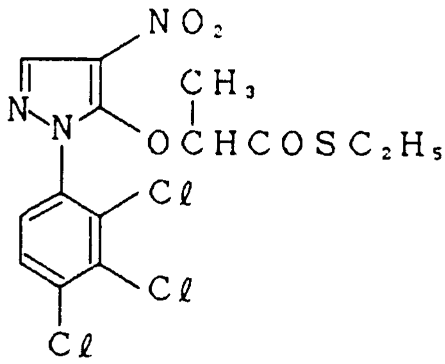

Example 7

Preparation of [4-nitro-1-(2,3,4-trichlorophenyl)pyrazole-5-yl]-2-propionic acid benzyl amide

In 20ml of benzene was dissolved [4-nitro-1-(2,3,4-trichlorophenyl)pyrazole-5-yl]-2-propionyl chloride obtained in the same manner as in Example 6. To the solution was added 0.6g of benzylamine with ice-cooling and stirring and 0.5g of pyridine was added dropwise thereto. After stirring at room temperature for 1 hour, the mixture was washed with 2N hydrochloric acid aqueous solution, water and saturated sodium chloride aqueous solution, and dried over anhydrous magnesium sulfate. The solvent was removed at a reduced pressure and the resulting oil was purified by silica gel column chromatography to obtain 0.4g of the desired product.

m. p. 87.8 °C

NMR δppm ($CDCl_3$)

1.44 (d, 3H)

4.28 (d, 2H)

5.08 (q, 1H)

6.76 (broad, 1H)

6.8~7.5 (7H, aromatic ring)

8.03 (s, 1H)

| Elementary analysis ($C_{19}H_{15}N_4O_4Cl_3$ : 469.72) | | | |
|---|---|---|---|
| Calcd (%) | C : 48.58 | H : 3.22 | N : 11.93 |
| Found (%) | C : 48.21 | H : 3.56 | N : 12.06 |

From the above, the following compound was confirmed.

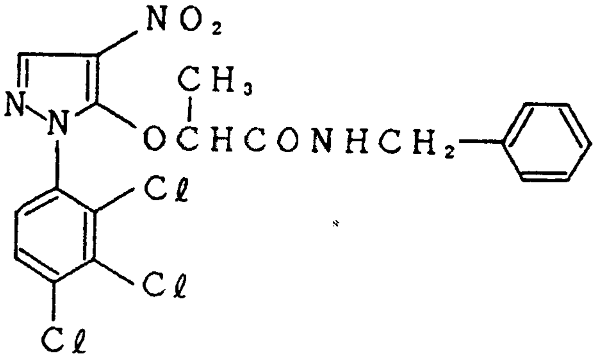

Examples 8 to 41

The present compounds listed in the following Table 1 were prepared in the same manner as in Examples 1 to 7. The property and NMR data were shown also in Table 1.

Table 1

| Ex. | Formula | Property |
|---|---|---|
| 8 | 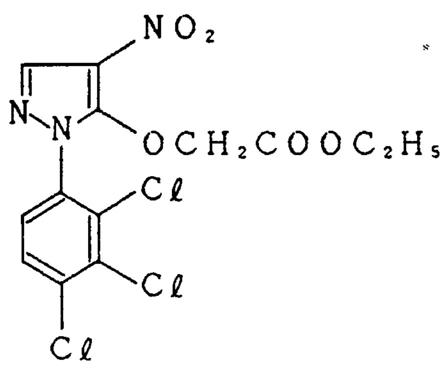 | Oil<br>NMR($CDCl_3$)<br>$\delta$ ppm<br>1.21(t, 3 H)<br>4.10(q, 2 H)<br>5.06(s, 2 H)<br>7.1~7.7(m, 2 H)<br>8.06(s, 1 H) |
| 9 | 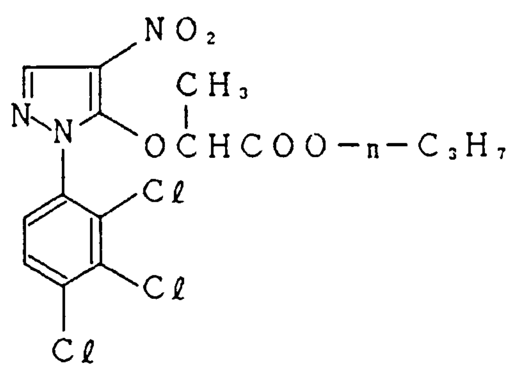 | Oil<br>NMR($CDCl_3$)<br>$\delta$ ppm<br>0.86(t, 3 H)<br>1.55(d, 3 H)<br>1.0~2.0(m, 2 H)<br>3.95(t, 2 H)<br>5.49(q, 1 H)<br>7.0~7.8(m, 2 H)<br>8.04(s, 1 H) |
| 10 | 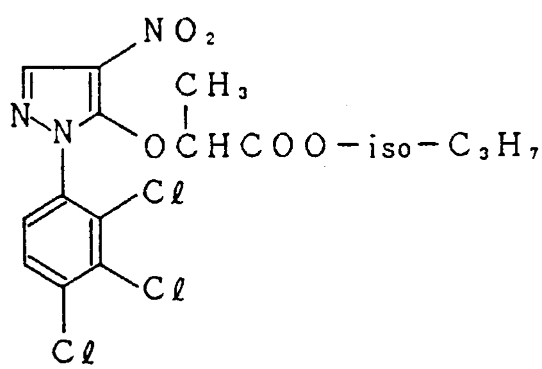 | Oil<br>NMR($CDCl_3$)<br>$\delta$ ppm<br>1.14(d, 6 H)<br>1.49(d, 3 H)<br>4.5~5.2(m, 1 H)<br>5.43(q, 1 H)<br>7.0~7.7(m, 2 H)<br>8.00(s, 1 H) |

Table 1 (continued)

| Ex. | Formula | Property |
|---|---|---|
| 11 | 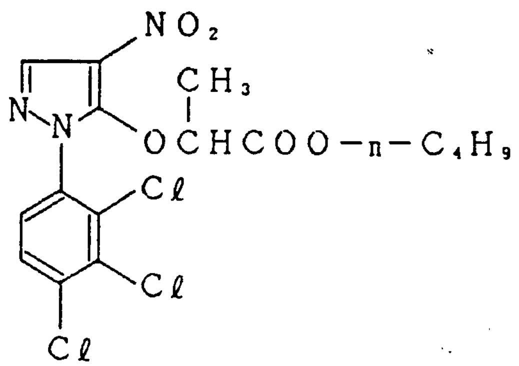 | Oil<br>NMR($CDCl_3$)<br>δ ppm<br>0.6~2.0(m, 7 H)<br>1.55(d, 3 H)<br>3.98(t, 2 H)<br>5.48(q, 1 H)<br>7.0~7.6(m, 2 H)<br>8.04(s, 1 H) |
| 12 | 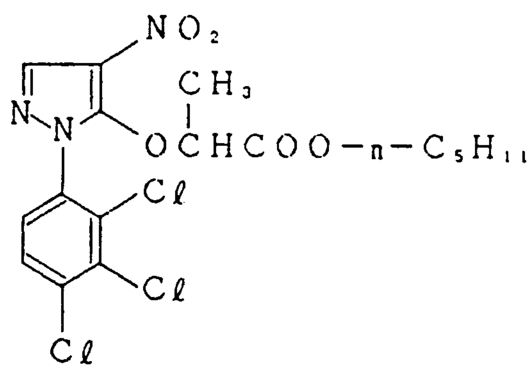 | Oil<br>NMR($CDCl_3$)<br>δ ppm<br>0.5~2.0(m, 9 H)<br>1.55(d, 3 H)<br>3.96(t, 2 H)<br>5.47(q, 1 H)<br>7.0~7.6(m, 2 H)<br>8.01(s, 1 H) |
| 13 | 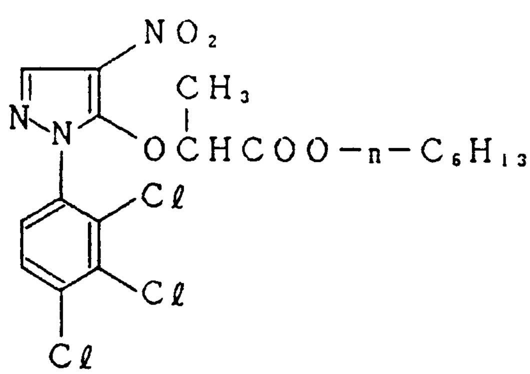 | Oil<br>NMR($CDCl_3$)<br>δ ppm<br>0.5~2.0(m, 11H)<br>1.53(d, 3 H)<br>3.96(t, 2 H)<br>5.46(q, 1 H)<br>7.0~7.6(m, 2 H)<br>8.00(s, 1 H) |

Table 1 (continued)

| Ex. | Formula | Property |
|---|---|---|
| 14 | 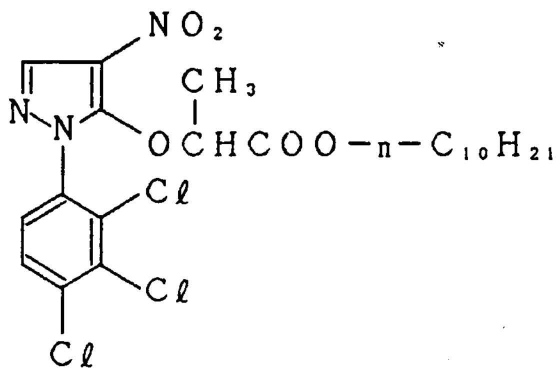 | Oil<br>NMR($CDC\ell_3$)<br>δ ppm<br>0.5～2.0(m, 19H)<br>1.53(d, 3H)<br>3.98(t, 2H)<br>5.49(q, 1H)<br>7.0～7.7(m, 2H)<br>8.06(s, 1H) |
| 15 | 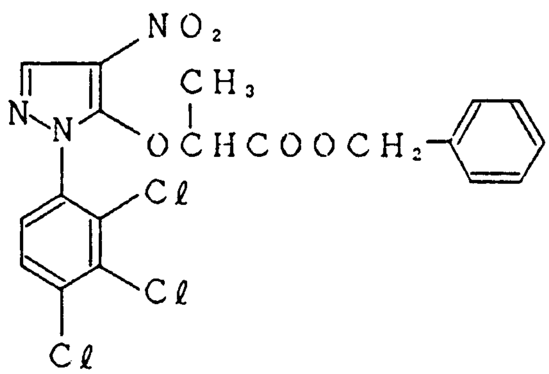 | Oil<br>NMR($CDC\ell_3$)<br>δ ppm<br>1.55(d, 3H)<br>4.98(s, 2H)<br>5.53(q, 1H)<br>6.8～7.6(m, 7H)<br>8.01(s, 1H) |
| 16 | 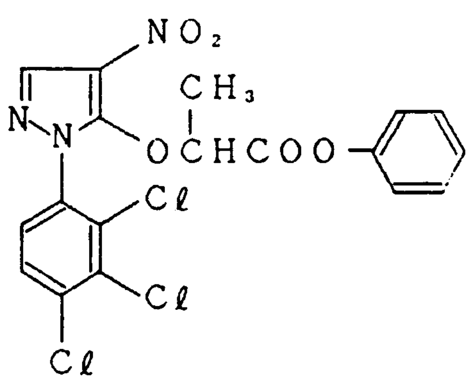 | m.p. 83～84℃<br>NMR($CDC\ell_3$)<br>δ ppm<br>1.71(d, 3H)<br>5.72(q, 1H)<br>6.5～7.6(m, 7H)<br>8.07(s, 1H) |

Table 1 (continued)

| Ex. | Formula | Property |
|---|---|---|
| 17 | 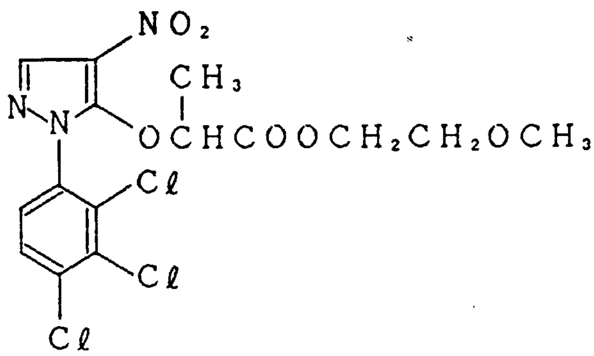 | Oil<br>NMR($CDCl_3$)<br>δ ppm<br>1.55(d, 3 H)<br>3.25(s, 3 H)<br>3.2~3.8(m, 2 H)<br>3.9~4.4(m, 2 H)<br>5.52(q, 1 H)<br>7.1~7.7(m, 2 H)<br>8.05(s, 1 H) |
| 18 | 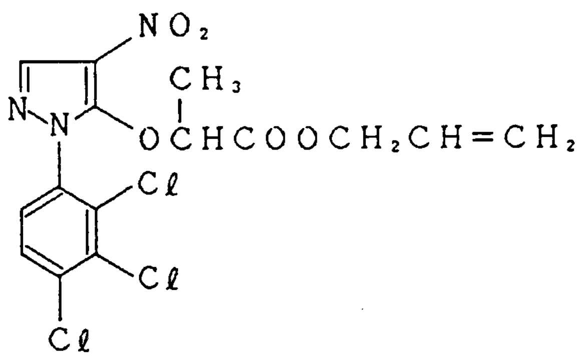 | Oil<br>NMR($CDCl_3$)<br>δ ppm<br>1.55(d, 3 H)<br>4.45(d, 2 H)<br>4.8~6.2(m, 4 H)<br>7.1~7.6(m, 2 H)<br>8.02(s, 1 H) |
| 19 | 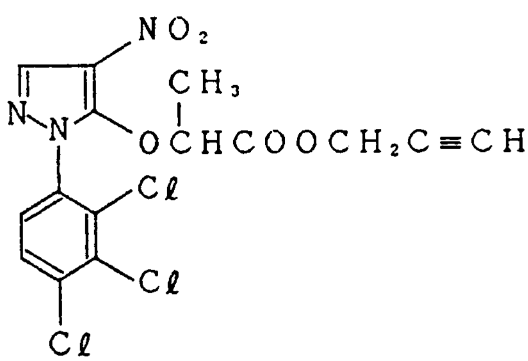 | Oil<br>NMR($CDCl_3$)<br>δ ppm<br>1.58(d, 3 H)<br>2.45(t, 1 H)<br>4.55(d, 2 H)<br>5.50(q, 1 H)<br>7.0~7.7(m, 2 H)<br>8.05(s, 1 H) |

Table 1 (continued)

| Ex. | Formula | Property |
|---|---|---|
| 20 | 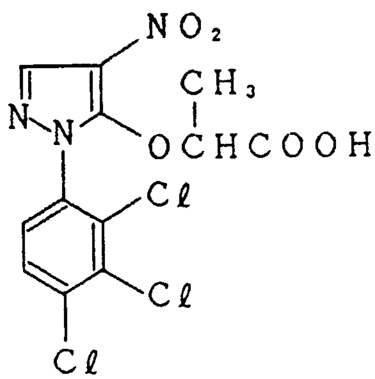 | m. p. 115~116℃<br>NMR($CDCl_3$)<br>δ ppm<br>1.54(d, 3H)<br>5.48(q, 1H)<br>7.1~7.7(m, 2H)<br>8.07(s, 1H) |
| 21 | 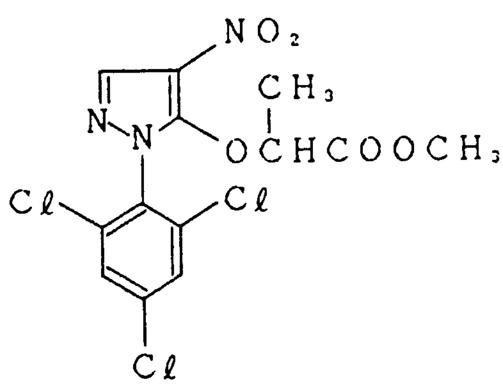 | m. p. 186~187℃<br>NMR($CDCl_3$)<br>δ ppm<br>1.55(d, 3H)<br>3.60(s, 3H)<br>7.3~7.5(m, 2H)<br>8.09(s, 1H) |
| 22 | 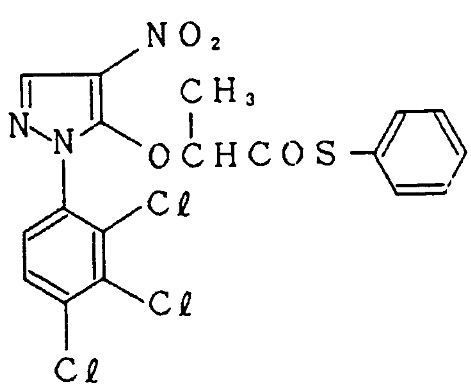 | m.p. 76~77℃<br>NMR($CDCl_3$)<br>δ ppm<br>1.56(d, 3H)<br>5.55(q, 1H)<br>7.7~7.5(m, 7H)<br>8.04(s, 1H) |

Table 1 (continued)

| Ex. | Formula | Property |
|---|---|---|
| 23 | 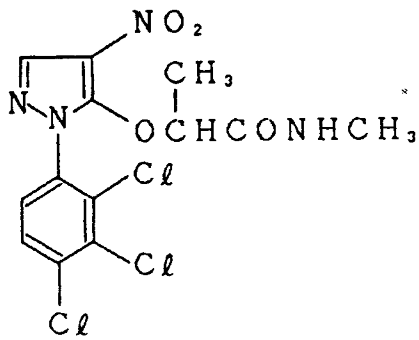 | m.p. 136~137℃<br>NMR(CDCℓ₃)<br>δ ppm<br>1.35(d, 3 H)<br>2.73(d, 3 H)<br>4.99(q, 1 H)<br>6.50(s, 1 H)<br>7.0~7.6(m, 2 H)<br>8.11(s, 1 H) |
| 24 | 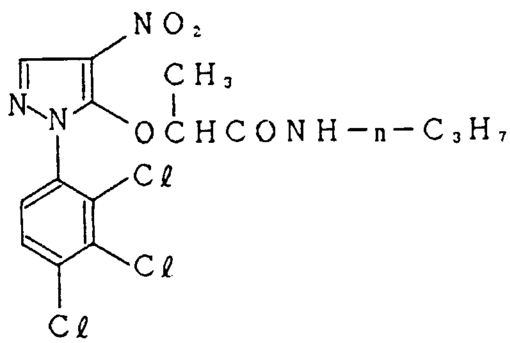 | Oil<br>NMR(CDCℓ₃)<br>δ ppm<br>0.5~2.0(m, 5 H)<br>1.35(d, 3 H)<br>3.12(q, 2 H)<br>4.98(q, 1 H)<br>6.40(s, 1 H)<br>7.0~7.7(m, 2 H)<br>8.07(s, 1 H) |
| 25 | 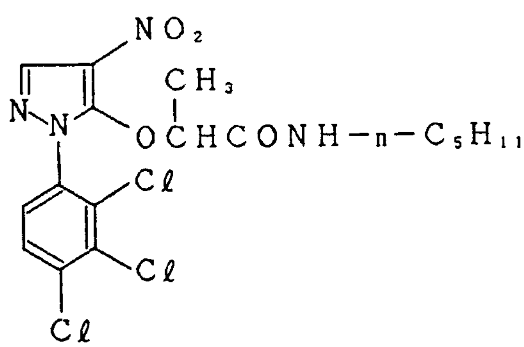 | Oil<br>NMR(CDCℓ₃)<br>δ ppm<br>0.87(t, 3 H)<br>1.0~2.0(m, 6 H)<br>1.36(d, 3 H)<br>3.10(q, 2 H)<br>5.03(q, 1 H)<br>6.56(s, 1 H)<br>7.1~7.7(m, 2 H)<br>8.07(s, 1 H) |

Table 1 (continued)

| Ex. | Formula | Property |
|---|---|---|
| 26 | 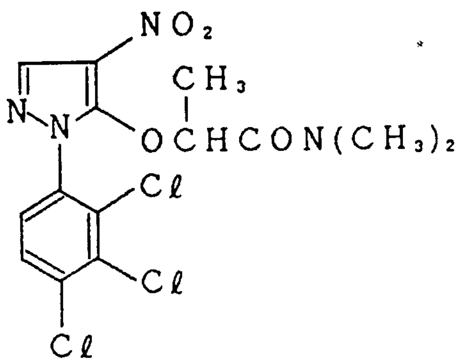 | m.p. 139~140℃<br>NMR($CDCl_3$)<br>δ ppm<br>1.40(d, 3 H)<br>2.81(s, 3 H)<br>2.94(s, 3 H)<br>6.03(q, 1 H)<br>7.1~7.8(m, 2 H)<br>8.01(s, 1 H) |
| 27 | 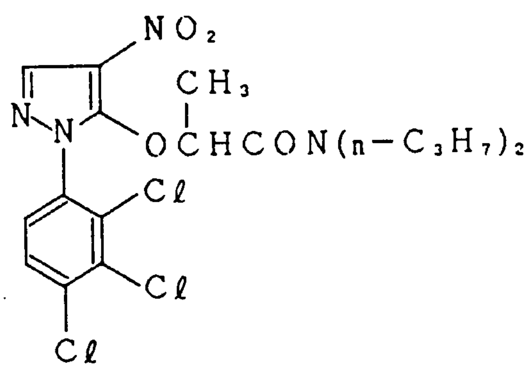 | m.p. 101~102℃<br>NMR($CDCl_3$)<br>δ ppm<br>0.5~2.0(m,10H)<br>1.39(d, 3 H)<br>3.16(t, 4 H)<br>6.00(q, 1 H)<br>7.0~7.7(m, 2 H)<br>8.03(s, 1 H) |
| 28 | 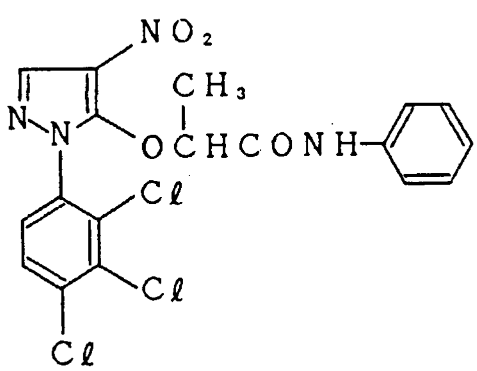 | Oil<br>NMR($CDCl_3$)<br>δ ppm<br>1.40(d, 3 H)<br>5.02(q, 1 H)<br>6.5~7.5(m, 2 H)<br>8.06(s, 1 H)<br>8.25(s, 1 H) |

Table 1 (continued)

| Ex. | Formula | Property |
|---|---|---|
| 29 | 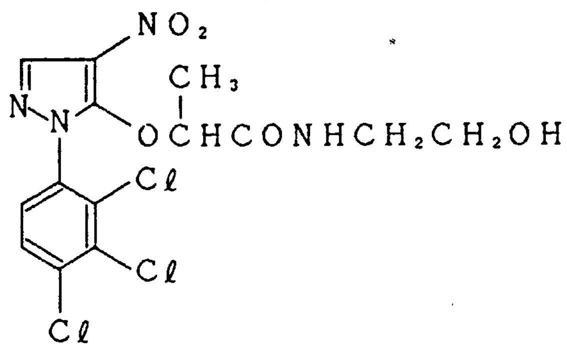 | Oil<br>NMR($CDCl_3$)<br>δ ppm<br>1.34(d, 3 H)<br>3.0~4.5(m, 4 H)<br>5.04(q, 1 H)<br>7.1~7.7(m, 2 H)<br>8.06(s, 1 H) |
| 30 | 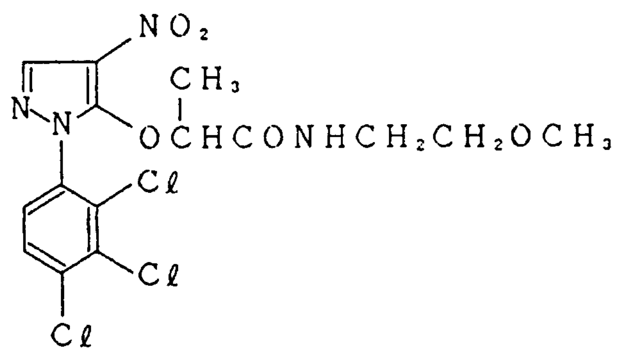 | m.p. 83~84℃<br>NMR($CDCl_3$)<br>δ ppm<br>1.37(d, 3 H)<br>3.27(s, 3 H)<br>3.1~3.6(m, 4 H)<br>5.09(q, 1 H)<br>6.65(s, 1 H)<br>7.1~7.7(m, 2 H)<br>8.09(s, 1 H) |
| 31 | 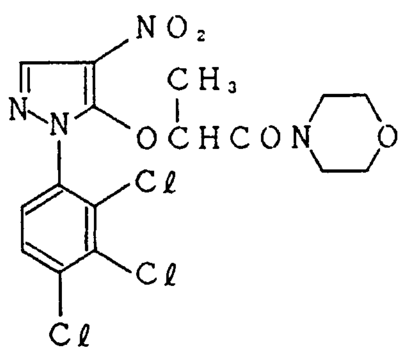 | m.p. 143~144℃<br>NMR($CDCl_3$)<br>δ ppm<br>1.40(d, 3 H)<br>3.1~3.9(m, 8 H)<br>6.04(q, 1 H)<br>7.2~7.7(m, 2 H)<br>8.04(s, 1 H) |

Table 1 (continued)

| Ex. | Formula | Property |
|---|---|---|
| 32 | 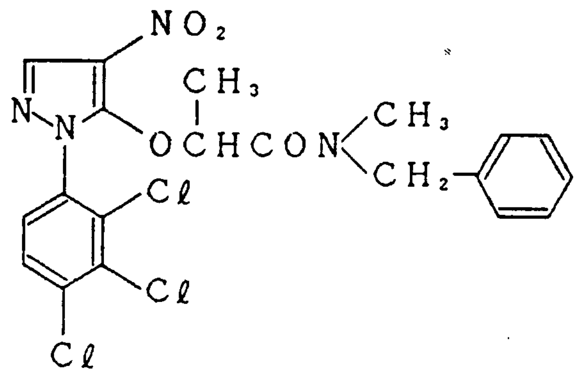 | Oil<br>NMR($CDCl_3$)<br>δ ppm<br>1.44(d, 3 H)<br>2.83(s, 3 H)<br>4.40(dd, 2 H)<br>6.10(q, 1 H)<br>6.8~7.8(m, 7 H)<br>8.01(s, 1 H) |
| 33 | 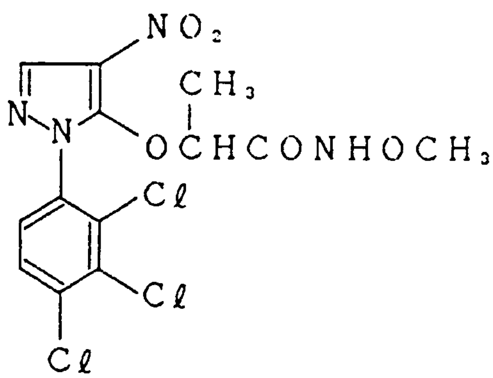 | Oil<br>NMR($CDCl_3$)<br>δ ppm<br>1.33(d, 3 H)<br>2.94(s, 1 H)<br>3.60(s, 3 H)<br>4.50(q, 1 H)<br>7.0~7.6(m, 2 H)<br>8.29(s, 1 H) |
| 34 | 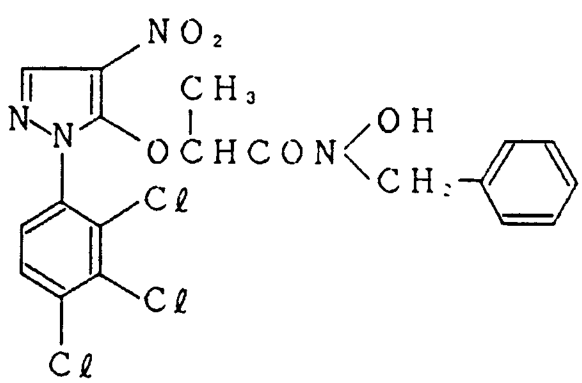 | Oil<br>NMR($CDCl_3$)<br>δ ppm<br>1.36(d, 3 H)<br>4.54(s, 2 H)<br>5.92(q, 1 H)<br>6.8~7.6(m, 7 H)<br>7.85(s, 1 H) |

Table 1 (continued)

| Ex. | Formula | Property |
|---|---|---|
| 35 | 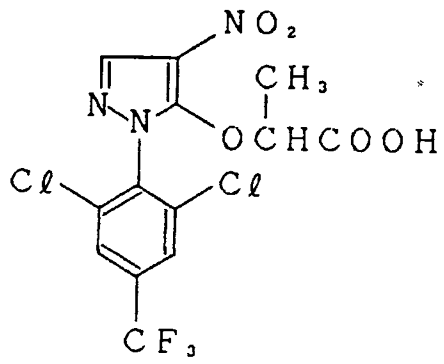 | Oil<br>NMR($CDCℓ_3$)<br>δ ppm<br>1.57(d, 3 H)<br>5.35(q, 1 H)<br>7.55(s, 2 H)<br>8.07(s, 1 H) |
| 36 | 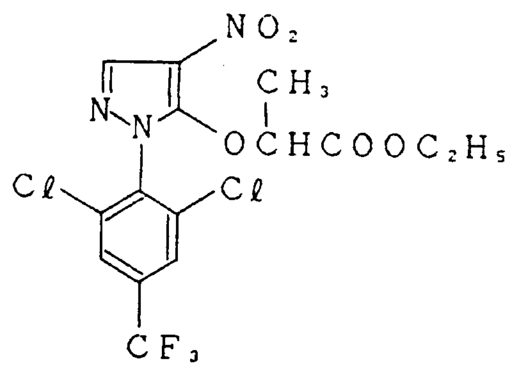 | Oil<br>NMR($CDCℓ_3$)<br>δ ppm<br>1.18(t, 3 H)<br>1.54(d, 3 H)<br>4.03(q, 2 H)<br>5.35(q, 1 H)<br>7.62(s, 2 H)<br>8.10(s, 1 H) |
| 37 | 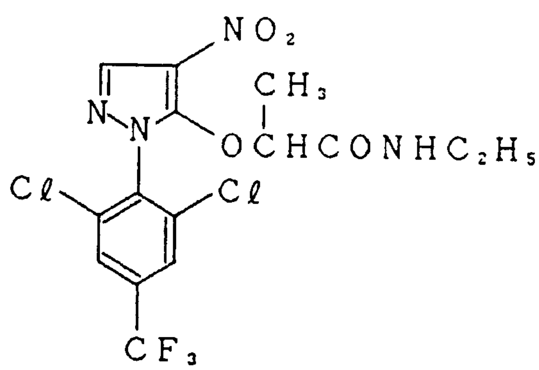 | Oil<br>NMR($CDCℓ_3$)<br>δ ppm<br>1.38(d, 3 H)<br>3.22(q, 2 H)<br>4.96(q, 1 H)<br>6.38(s, 1 H)<br>7.67(s, 2 H)<br>8.14(s, 1 H) |

Table 1 (continued)

| Ex. | Formula | Property |
|---|---|---|
| 38 | 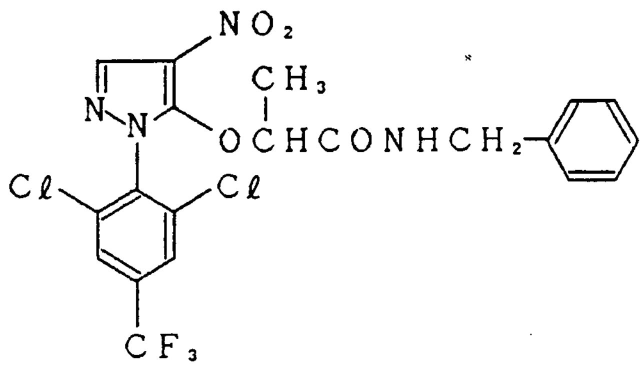 | Oil<br>NMR($CDC\ell_3$)<br>δ ppm<br>1.39(d, 3 H)<br>4.31(d, 2 H)<br>5.11(q, 1 H)<br>6.68(t, 1 H)<br>7.12(s, 5 H)<br>7.57(d, 2 H)<br>8.13(s, 1 H) |
| 39 | 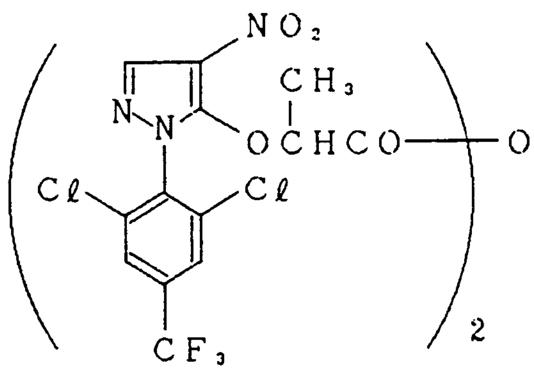 | m.p. 114～115℃<br>NMR($CDC\ell_3$)<br>δ ppm<br>1.18(d, 6 H)<br>4.77(q, 2 H)<br>7.62(s, 4 H)<br>7.72(s, 2 H) |
| 40 | 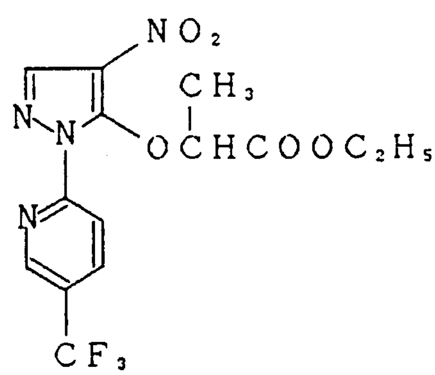 | Oil<br>NMR($CDC\ell_3$)<br>δ ppm<br>1.13(t, 3 H)<br>1.71(d, 3 H)<br>4.02(q, 2 H)<br>5.43(q, 1 H)<br>8.10(s, 1 H)<br>7.5～9.0(m, 3 H) |

Table 1 (continued)

| Ex. | Formula | Property |
|---|---|---|
| 41 | 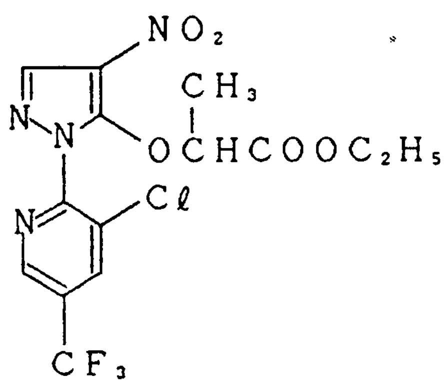 | Oil<br>NMR($CDCl_3$)<br>δ ppm<br>1.17(t, 3 H)<br>1.60(d, 3 H)<br>4.02(q, 2 H)<br>5.35(q, 1 H)<br>8.12(s, 1 H)<br>8.0~9.0(m, 2 H) |

Herbicide Preparation 1 (Emulsion)

| | |
|---|---|
| The present compound | 30 (wt. parts) |
| polyoxyethylene alkylphenyl ether | 12 |
| sodium alkylbenzene sulfonate | 3 |
| xylene | 55 |
| | 100 |

The above components were thoroughly mixed with stirring to obtain a 30% emulsion.

Herbicide Preparation 2 (Wettable powder)

| | |
|---|---|
| The present compound | 30 (wt. parts) |
| white carbon | 40 |
| kieselguhr | 15 |
| sodium lauryl sulfate | 3 |
| sodium 2,2'-dinaphthylmethanesulfonate | 2 |
| alkylphenylphenol sulfuric acid salt | 10 |
| | 100 |

The above components were thoroughly admixed by use of a mixer and finely pulverized with use of a mill to prepare a 30% wettable powder.

Herbicide Preparation 3 (Granules)

| | |
|---|---|
| The present compound | 5 (wt. parts) |
| synthetic hydrated silicone oxide | 1 |
| calcium lignin sulfonate | 2 |
| bentonite | 25 |
| kaolin clay | 67 |
| | 100 |

The above components were thoroughly pulverized and mixed. The mixture was admixed with addition of water, made into granules and dried to prepare 5% granules.

Test Example 1

Treatment under submerged condition

A pot (200cm$^2$) was filled with paddy soil, and several plants were sown or transplanted. Tubers of Sagittaria pygmaea were mixed in the surface 1cm of the soil. About 50 seeds of annual weeds such as barnyardgrass, toothcup, falsepimpernel, Monochoria vaginalis, and flatsedge were sown. Rice seedling (2-leaf stage) were transplanted 2.5cm deep. A pot was irrigated and kept covered with 3cm of water thereafter. Three days after transplanting rice seedlings, the wettable powder prepared in Herbicide Preparation 2 was added to the water at 0.15, 0.3 and 0.6kg ai/ha. The treated pots were maintained in the green house. One month after treatment, herbicidal effects and injury against transplanted rice seedlings were assessed. The results are shown in Table 2. The criterion for examination was followed as described below.

Index for herbicidal activity:

| | |
|---|---|
| 5 | complete kill |
| 4 | approximately 80% kill |
| 3 | approximately 60% kill |
| 2 | approximately 40% kill |
| 1 | approximately 20% kill |
| 0 | no effect |

Index for chemical injury:

| | |
|---|---|
| - | no injury |
| + | slight injury |
| + + | a little injury |
| + + + | serious injury |
| + + + + | severe injury |
| X | death |

Table 2

| Compd. No. | Amount kg ai/ha | Herbicidal effect barnyard-grass | broad-leaves | flatsedge | Sagittaria pygmaea | Injury against rice |
|---|---|---|---|---|---|---|
| | 0.15 | 5 | 5 | 5 | 5 | − |
| 2 | 0.30 | 5 | 5 | 5 | 5 | + |
| | 0.60 | 5 | 5 | 5 | 5 | + |
| | 0.15 | 5 | 5 | 5 | 5 | − |
| 4 | 0.30 | 5 | 5 | 5 | 5 | − |
| | 0.60 | 5 | 5 | 5 | 5 | + |
| | 0.15 | 5 | 5 | 5 | 4 | − |
| 6 | 0.30 | 5 | 5 | 5 | 5 | − |
| | 0.60 | 5 | 5 | 5 | 5 | + |
| | 0.15 | 5 | 5 | 4 | 4 | − |
| 7 | 0.30 | 5 | 5 | 5 | 5 | − |
| | 0.60 | 5 | 5 | 5 | 5 | − |
| | 0.15 | 3 | 3 | 2 | 2 | − |
| 8 | 0.30 | 3 | 4 | 3 | 2 | − |
| | 0.60 | 4 | 4 | 3 | 3 | − |
| | 0.15 | 4 | 4 | 3 | 3 | − |
| 13 | 0.30 | 5 | 4 | 4 | 4 | − |
| | 0.60 | 5 | 5 | 4 | 4 | − |
| | 0.15 | 4 | 3 | 3 | 3 | − |
| 16 | 0.30 | 4 | 4 | 3 | 3 | − |
| | 0.60 | 5 | 4 | 4 | 4 | − |
| | 0.15 | 3 | 3 | 2 | 2 | − |
| 21 | 0.30 | 3 | 3 | 3 | 2 | − |
| | 0.60 | 4 | 4 | 4 | 3 | − |
| | 0.15 | 5 | 4 | 4 | 3 | − |
| 23 | 0.30 | 5 | 4 | 4 | 4 | − |
| | 0.60 | 5 | 5 | 5 | 4 | − |

Table 2 (continued)

| Compd. No. | Amount kg ai/ha | Herbicidal effect barnyard -grass | broad-leaves | flatsedge | Sagittaria pygmaea | Injury against rice |
|---|---|---|---|---|---|---|
| 30 | 0.15 | 5 | 5 | 4 | 4 | − |
| | 0.30 | 5 | 5 | 4 | 4 | − |
| | 0.60 | 5 | 5 | 5 | 5 | + |
| 36 | 0.15 | 5 | 5 | 5 | 5 | − |
| | 0.30 | 5 | 5 | 5 | 5 | + |
| | 0.60 | 5 | 5 | 5 | 5 | + |
| 38 | 0.15 | 5 | 5 | 5 | 5 | − |
| | 0.30 | 5 | 5 | 5 | 5 | − |
| | 0.60 | 5 | 5 | 5 | 5 | − |
| 40 | 0.15 | 4 | 3 | 3 | 3 | − |
| | 0.30 | 4 | 4 | 3 | 3 | − |
| | 0.60 | 5 | 5 | 4 | 4 | − |
| 41 | 0.15 | 5 | 4 | 4 | 4 | − |
| | 0.30 | 5 | 5 | 5 | 5 | − |
| | 0.60 | 5 | 5 | 5 | 5 | + |

Test Example 2

Preemergence treatment

Test compounds (Herbicide Preparation 2) were treated at 0.6 and 1.2 kg ai/ha. Test plants are shown in Table 3. Twenty-one days after treatment, herbicidal effects were assembled. The results are shown in Table 3.

Table 3

| Compd. No. | Amount kg ai/ha | Herbicidal effect: large crabgrass | foxtail | velvetleaf | redroot pigweed |
|---|---|---|---|---|---|
| 2 | 0.6 | 3 | 3 | 5 | 5 |
| | 1.2 | 4 | 4 | 5 | 5 |
| 4 | 0.6 | 3 | 3 | 5 | 5 |
| | 1.2 | 4 | 4 | 5 | 5 |
| 6 | 0.6 | 3 | 3 | 5 | 5 |
| | 1.2 | 3 | 3 | 5 | 5 |
| 7 | 0.6 | 3 | 3 | 5 | 5 |
| | 1.2 | 3 | 4 | 5 | 5 |
| 11 | 0.6 | 3 | 3 | 5 | 5 |
| | 1.2 | 4 | 3 | 5 | 5 |
| 19 | 0.6 | 3 | 3 | 5 | 5 |
| | 1.2 | 4 | 4 | 5 | 5 |
| 26 | 0.6 | 3 | 3 | 5 | 5 |
| | 1.2 | 4 | 3 | 5 | 5 |
| 36 | 0.6 | 5 | 5 | 5 | 5 |
| | 1.2 | 5 | 5 | 5 | 5 |
| 37 | 0.6 | 5 | 5 | 5 | 5 |
| | 1.2 | 5 | 5 | 5 | 5 |
| 38 | 0.6 | 5 | 4 | 5 | 5 |
| | 1.2 | 5 | 5 | 5 | 5 |
| 39 | 0.6 | 4 | 4 | 5 | 5 |
| | 1.2 | 5 | 4 | 5 | 5 |
| 40 | 0.6 | 3 | 3 | 5 | 5 |
| | 1.2 | 4 | 3 | 5 | 5 |
| 41 | 0.6 | 5 | 5 | 5 | 5 |
| | 1.2 | 5 | 5 | 5 | 5 |

Test Example 3

Postemergence treatment

Test compounds (Herbicide preparation 2) was treated at 0.3 and 0.6 kg ai/ha. Test plants (2~3 weeks old-plants, 2~4-leaf stage) are shown in Table 4. Twenty-one days after treatment, herbicidal effects were assessed. The results are shown in Table 4.

Table 4

| Compd. No. | Amount kg ai/ha | Herbicidal effect: large crabgrass | foxtail | velvetleaf | redroot pigweed |
|---|---|---|---|---|---|
| 2 | 0.3 | 5 | 5 | 5 | 5 |
| | 0.6 | 5 | 5 | 5 | 5 |
| 4 | 0.3 | 5 | 5 | 5 | 5 |
| | 0.6 | 5 | 5 | 5 | 5 |
| 6 | 0.3 | 5 | 5 | 5 | 5 |
| | 0.6 | 5 | 5 | 5 | 5 |
| 10 | 0.3 | 5 | 5 | 5 | 5 |
| | 0.6 | 5 | 5 | 5 | 5 |
| 15 | 0.3 | 4 | 4 | 5 | 5 |
| | 0.6 | 5 | 5 | 5 | 5 |
| 23 | 0.3 | 4 | 3 | 5 | 5 |
| | 0.6 | 5 | 4 | 5 | 5 |
| 31 | 0.3 | 4 | 3 | 5 | 5 |
| | 0.6 | 4 | 4 | 5 | 5 |
| 36 | 0.3 | 4 | 4 | 5 | 5 |
| | 0.6 | 5 | 4 | 5 | 5 |
| 38 | 0.3 | 3 | 3 | 5 | 5 |
| | 0.6 | 4 | 4 | 5 | 5 |

Table 4

| Compd. No. | Amount kg ai/ha | Herbicidal effect | | | |
|---|---|---|---|---|---|
| | | large crabgrass | foxtail | velvetleaf | redroot pigweed |
| 40 | 0.3 | 3 | 2 | 5 | 5 |
| | 0.6 | 4 | 3 | 5 | 5 |
| 41 | 0.3 | 4 | 3 | 5 | 5 |
| | 0.6 | 4 | 4 | 5 | 5 |

## Claims

1. A pyrazole derivative of the formula

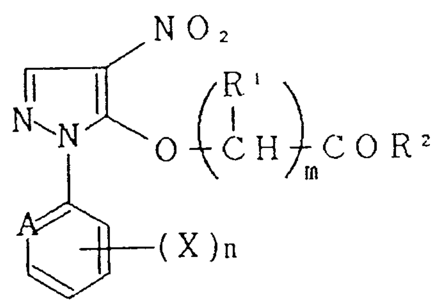

wherein $R^1$ is hydrogen or lower $C_1$-$C_6$ alkyl, $R^2$ is hydroxyl, $-OR^3$, $-SR^3$ or $-NR^4R^5$, $R^3$ is $C_1$-$C_{12}$ alkyl, lower $C_2$-$C_6$ alkenyl, lower $C_2$-$C_6$ alkynyl, $C_3$-$C_8$ cycloalkyl, lower $C_2$-$C_8$ alkoxyalkyl, phenyl optionally substituted with halogen, $C_1$-$C_3$ alkyl, $C_1$-$C_3$ alkoxyl, nitro or cyano, or benzyl optionally substituted with halogen, $C_1$-$C_3$ alkyl, $C_1$-$C_3$ alkoxyl, nitro or cyano, $R^4$ and $R^5$ are the same or different and are hydrogen, lower $C_1$-$C_6$ alkyl, lower $C_2$-$C_6$ alkenyl, lower $C_2$-$C_6$ alkynyl, hydroxyl, lower $C_1$-$C_6$ alkoxyl, lower $C_1$-$C_6$ alkyl having substituent(s) selected from halogen, cyano, hydroxyl, and alkoxyl, $C_3$-$C_8$ cycloalkyl, phenyl optionally substituted with halogen, $C_1$-$C_3$ alkyl, $C_1$-$C_3$ alkoxyl, nitro or cyano, or benzyl optionally substituted with halogen, $C_1$-$C_3$ alkyl, $C_1$-$C_3$ alkoxyl, nitro or cyano, $R^4$ and $R^5$ may link together to form a 5 or 6-membered ring, X is halogen, trifluoromethyl, trifluoromethoxy or trifluoromethylthio, ⋟A represents ⋟CH or ⋟N, m is 1 or 2, n is an integer of 1 to 5.

2. A process for preparing a pyrazole derivative of claim 1 which comprises reacting a pyrazole derivative of the formula

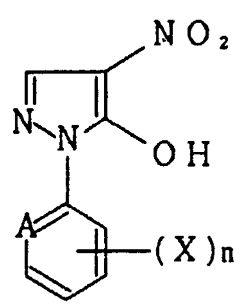

with a compound of the formula

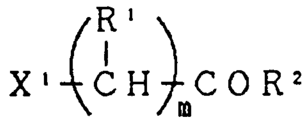

wherein $R^1$, $R^2$, X, m and n are the same as above, $X^1$ is halogen atom.

**3.** A process for preparing a pyrazole derivative of claim 1 which comprises nitrating a pyrazole derivative of the formula

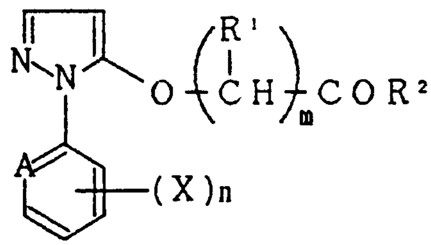

wherein $R^1$, $R^2$, X, m and n are the same as above.

**4.** A herbicide containing as an effective component a pyrazole derivative of claim 1.

**Patentansprüche**

**1.** Pyrazolderivat der Formel

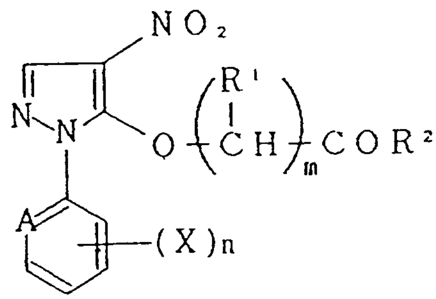

worin $R^1$ Wasserstoff oder Nieder-$C_1$-$C_6$-alkyl bedeutet, $R^2$ Hydroxyl, -$OR^3$, -$SR^3$ oder -$NR^4R^5$ darstellt, $R^3$ für $C_1$-$C_{12}$-Alkyl, Nieder-$C_2$-$C_6$-alkenyl, Nieder-$C_2$-$C_6$-alkinyl, $C_3$-$C_8$-Cycloalkyl, Nieder-$C_2$-$C_8$-alkoxyalkyl, gegebenenfalls mit Halogen, $C_1$-$C_3$-Alkyl, $C_1$-$C_3$-Alkoxy, Nitro oder Cyano substituiertes Phenyl oder gegebenenfalls mit Halogen, $C_1$-$C_3$-Alkyl, $C_1$-$C_3$-Alkoxy, Nitro oder Cyano substituiertes Benzyl steht, $R^4$ und $R^5$ gleich oder verschieden sind und Wasserstoff, Nieder-$C_1$-$C_6$-alkyl, Nieder-$C_2$-$C_6$-alkenyl, Nieder-$C_2$-$C_6$-alkinyl, Hydroxyl, Nieder-$C_1$-$C_6$-alkoxy, Nieder-$C_1$-$C_6$-alkyl mit Substituent(en), der (die) ausgewählt ist (sind) aus Halogen, Cyano, Hydroxyl und Alkoxy, $C_3$-$C_8$-Cycloalkyl, gegebenenfalls mit Halogen, $C_1$-$C_3$-Alkyl, $C_1$-$C_3$-Alkoxy, Nitro oder Cyano substituiertes Phenyl oder gegebenenfalls mit Halogen, $C_1$-$C_3$-Alkyl, $C_1$-$C_3$-Alkoxy, Nitro oder Cyano substituiertes Benzyl darstellen, $R^4$ und $R^5$ sich zusammenbinden können, um einen 5- oder 6-gliedrigen Ring zu bilden, X für Halogen, Trifluormethyl, Trifluormethoxy oder Trifluormethylthio steht, ⩾A ⩾CH oder ⩾N repräsentiert, m 1 oder 2 ist, n eine ganze Zahl von 1 bis 5 darstellt.

**2.** Verfahren zur Herstellung eines Pyrazolderivats nach Anspruch 1, welches umfaßt die Umsetzung eines Pyrazolderivats der Formel

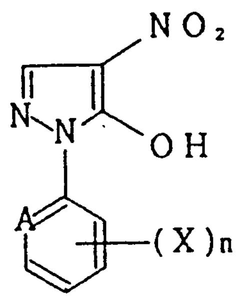

mit einer Verbindung der Formel

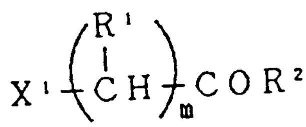

worin $R^1$, $R^2$, X, m und n gleich wie oben sind, $X^1$ ein Halogenatom ist.

**3.** Verfahren zur Herstellung eines Pyrazolderivats nach Anspruch 1, welches umfaßt die Nitrierung eines Pyrazolderivats der Formel

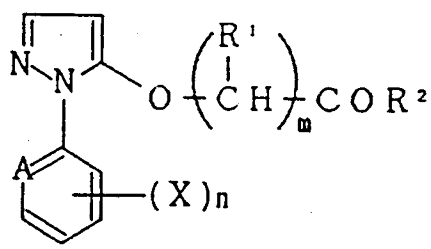

worin $R^1$, $R^2$, X, m und n gleich wie oben sind.

**4.** Herbizid, enthaltend als wirksame Komponente ein Pyrazolderivat nach Anspruch 1.

## Revendications

**1.** Dérivé du pyrazole, de formule

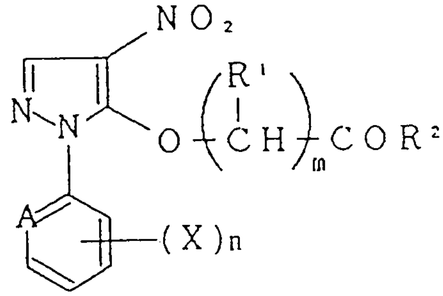

dans laquelle $R^1$ est un hydrogène ou un radical alkyle inférieur en $C_1$-$C_6$, $R^2$ est un radical hydroxyle, $-OR^3$, $-SR^3$ ou $-NR^4R^5$, $R^3$ est un radical alkyle en $C_1$-$C_{12}$, alcényle inférieur en $C_2$-$C_6$, alcynyle

inférieur en $C_2$-$C_6$, cycloalkyle en $C_3$-$C_8$, alcoxyalkyle inférieur en $C_2$-$C_8$, phényle éventuellement substitué par des substituants halogéno, alkyle en $C_1$-$C_3$, alcoxyle en $C_1$-$C_3$, nitro ou cyano, ou benzyle éventuellement substitué par des substituants halogéno, alkyle en $C_1$-$C_3$, alcoxyle en $C_1$-$C_3$, nitro ou cyano, $R^4$ et $R^5$ sont identiques ou différents et représentent chacun un hydrogène ou un radical alkyle inférieur en $C_1$-$C_6$, alcényle inférieur en $C_2$-$C_6$, alcynyle inférieur en $C_2$-$C_6$, hydroxyle, alcoxyle inférieur en $C_1$-$C_6$, alkyle inférieur en $C_1$-$C_6$ ayant un ou plusieurs substituants choisis parmi les substituants halogéno, cyano, hydroxy et alcoxy, cycloalkyle en $C_3$-$C_8$, phényle éventuellement substitué par des substituants halogéno, alkyle en $C_1$-$C_3$, alcoxyle en $C_1$-$C_3$, nitro ou cyano, ou benzyle éventuellement substitué par des substituants halogéno, alkyle en $C_1$-$C_3$, alcoxyle en $C_1$-$C_3$, nitro ou cyano, $R^4$ et $R^5$ peuvent être réunis pour former un noyau à 5 ou 6 chaînons, X est un halogène ou le radical trifluorométhyle, trifluorométhoxy ou trifluorométhylthio, A représente CH ou M, m vaut 1 ou 2 et n est un entier de 1 à 5.

**2.** Procédé pour préparer un dérivé du pyrazole selon la revendication 1, qui consiste à faire réagir un dérivé du pyrazole de formule

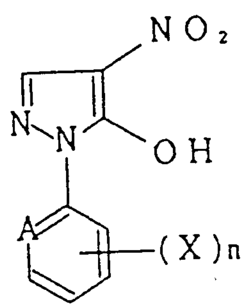

avec un composé de formule

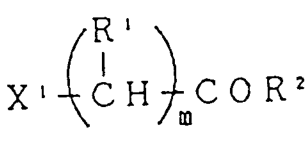

dans laquelle $R^1$, $R^2$, X, m et n sont les mêmes que ci-dessus et $X^1$ est un atome d'halogène.

**3.** Procédé pour préparer un dérivé du pyrazole selon la revendication 1, qui consiste à nitrer un dérivé du pyrazole de formule

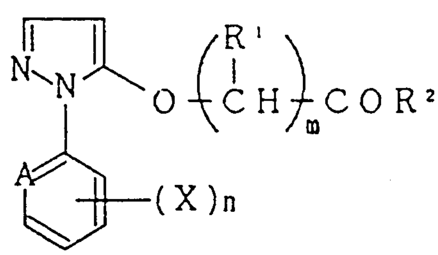

où $R^1$, $R^2$, X, m et n sont les mêmes que ci-dessus.

**4.** Herbicide contenant comme matière active un dérivé du pyrazole selon la revendication 1.